# EUROPÄISCHE PATENTSCHRIFT

(11) **EP 3 977 421 B1**
(45) Veröffentlichungstag und Bekanntmachung des Hinweises auf die Patenterteilung: **13.11.2024**
(21) Anmeldenummer: 21720689.5
(22) Anmeldetag: 18.03.2021
(51) Int. Cl.: G07C 9/20, G06F 21/31, G01N 33/48, G06F 21/32, C12Q 1/00, G06Q 50/22, C12Q 1/686, G07C 9/27, G07C 9/28, G16H 40/20

(54) **VERFAHREN UND SYSTEM ZUM INFEKTIONSSCHUTZ IN EINEM ZUGANGSBESCHRÄNKTEN BEREICH**
METHOD AND SYSTEM FOR PROTECTION AGAINST INFECTION IN AN AREA WITH RESTRICTED ACCESS
PROCÉDÉ ET SYSTÈME DE PROTECTION CONTRE UNE INFECTION DANS UNE ZONE À ACCÈS RESTREINT

(30) Priorität: 16.06.2020 DE 102020207446; 18.08.2020 DE 102020210490
(43) Veröffentlichungstag der Anmeldung: 06.04.2022
(73) Patentinhaber: HealthVision GmbH, 4312 Magden (CH)
(72) Erfinder: Fischer, Joachim, 69115 Heidelberg (DE)
(74) Vertreter: Ullrich & Naumann PartG mbB
(86) Internationale Anmeldenummer: PCT/DE2021/200036
(87) Internationale Veröffentlichungsnummer: WO 2021/254568

(56) Entgegenhaltungen:
- WO-A1-2011/005224
- US-A- 5 780 222
- US-A1- 2002 082 859
- US-A1- 2008 206 767

## Beschreibung

Die Erfindung betrifft ein System und ein Verfahren zum Infektionsschutz in einem zugangsbeschränkten oder zugangskontrollierbaren Bereich.

Während einer Epidemie oder einer Pandemie nimmt Infektionsschutz einen besonders hohen Stellenwert ein. Hierbei ist es wichtig, die Ausbreitung eines Krankheitserregers, meist eines Virus, möglichst effektiv einzudämmen. Ein Beispiel für ein derartiges Virus ist das SARS-CoV-2, das die Lungenkrankheit COVID-19 verursacht und sich Anfang 2020 global ausbreitete.

Besonders problematisch sind Krankheitserreger, bei denen eine Ansteckungsgefahr bereits besteht, bevor erste Krankheitssymptome auf eine Erkrankung hinweisen. So kann ein COVID-19-Patient bereits mehrere Tage vor Auftreten der ersten Krankheitszeichen andere Menschen anstecken. Für Personen mit frisch aufgetretenen Krankheitszeichen gilt, dass sie in der Regel um den Ausbruch der Krankheitssymptome die höchste Zahl an Viren ausscheiden und damit auch das höchste Ansteckungspotenzial aufweisen. Ferner verlaufen gerade bei COVID-19 zahlreiche Verläufe mit geringen Krankheitsanzeichen, häufig auch ohne Fieber. Damit kann auch eine (noch) asymptomatische Person andere Personen anstecken. Dies resultierte 2020 in einer hohen Reproduktionsrate R0 (Anzahl der von einer infizierten Person durchschnittlich angesteckten anderen Personen) von über 2,5. Die R0 war nicht für alle Infizierte gleich, sondern zeigte erhebliche Unterschiede zwischen Personen von kaum einer Ansteckung anderer bis zum "Superspreader". Die Unmöglichkeit, durch klassische Erkennungsmaßnahmen wie digitales Fiebermessen Infizierte zu erkennen, führte zusammen mit der enorm raschen und unerkannten Ausbreitung 2020 bei SARS-CoV-2 dazu, dass weltweit weite Teile des öffentlichen Lebens stillgelegt oder zumindest erheblich eingeschränkt wurden, um durch die weitgehende Kontaktsperre die Ausbreitung zu stoppen und wieder durch Nachverfolgung von Kontaktpersonen Infizierter beherrschbar zu machen.

Vielfach wird versucht, durch Ausgangssperren, Reiseverbote, Grenzschließungen, Schulschließungen, Home-Office, Abstandsregelungen, Hygienemaßnahmen, Nießetikette, Mund-Nase-Masken und andere Maßnahmen einen Infektionsschutz zu erzielen. In ihrer Gesamtheit waren diese Maßnahmen zwar wirksam, sind aber auf Dauer nicht vereinbar mit einer freiheitlichen Gesellschaft und freiem Wirtschaften. Das alleinige Tragen von Mund-Nase-Masken ist nachweislich nicht ausreichend und in vielen Bereichen wie etwa WellnessBereichen, Schwimmbädern oder Urlaubseinrichtungen ist das Tragen von Mund-Nase-Masken kaum oder überhaupt nicht möglich. Daher werden derartige Einrichtungen in der Praxis meist vollständig geschlossen oder mit erheblichen Restriktionen belegt. Dies stellt einerseits einen erheblichen wirtschaftlichen Schaden für die Betreiber derartiger Einrichtungen dar und führt andererseits zu einer starken Einschränkung betroffener Personen. Auch andere Bereiche mit potenzieller Zugangskontrolle, wie etwa Flughäfen, Flugzeuge, Schulen, Altenheime, Krankenhäuser oder Großanlässe (Sport, Konzerte), werden durch das reine Tragen von Mund-Nase-Masken allein unzureichend geschützt. An vielen dieser Orte lassen sich Abstandsregeln nur eingeschränkt umsetzen. Somit bleibt ein bedeutsames Restrisiko unerkannter erneuter Ausbreitung, sobald die strengen Schutzregeln gelockert werden.

Das Problem wäre deutlich beherrschbarer, stünde jeweils für den Bereich mit möglicher Zugangskontrolle eine tagesaktuelle Information zeitnah zur Verfügung, welche den Bereich betretende Person aktuell Viren verbreitet und damit andere Kontaktpersonen potenziell infiziert. Diese könnten bei entsprechender Koppelung mit anderen digitalen Systemen zur Identifikation des konkreten Aufenthaltsortes die potenziellen Kontaktpersonen stark eingrenzen und diese in Quarantäne versetzen, ehe sie weitere Personen anstecken. Selbst die Verbreitung durch einen "Superspreader" könnte so im Keim erstickt werden. Da die weit überwiegende Mehrzahl aller einen solchen Bereich betretenen Personen keine Viren verbreiten, wäre der Regelfall die umfassende Freigabe. Die klassische Untersuchung mit Nasen-Rachen-Abstrich und Einzeluntersuchung der Probe ist aber eine stark belästigende, und für eine Zeit des Abklingens der Pandemie unverhältnismäßig eingreifende, personalintensive und kostenintensive Methode.

Dokument US2002082859 A1 offenbart ein System in dem der Zutritt einer Person in einen Freizeitpark bezüglich des Gesundheitszustands der Person kontrolliert wird. Dokument US2008206767 A1 offenbart ein System in dem die Ausweitung einer Infektion in einem Krankenhaus beobachtet wird.

Der vorliegenden Erfindung liegt die Aufgabe zugrunde, ein System und ein Verfahren der eingangs genannten Art derart auszugestalten und weiterzubilden, dass ein Infektionsschutz in einem zugangsbeschränkten Bereich auch ohne erhebliche Restriktionen und zu wirtschaftliche vertretbaren Kosten möglich ist. Dabei ist erstrebenswert, wenn eine geringe Belästigung der den Bereich betretenden Personen und eine weitgehende Wahrung von Anonymität erreicht werden kann. Die Erfindung ist in den beigefügten Ansprüchen definiert.

Erfindungsgemäß wird die voranstehende Aufgabe durch die Merkmale des Anspruchs 1 gelöst. Danach umfasst das in Rede stehende System:
eine Testeinrichtung zum Testen einer Probe auf das Vorhandensein mindestens eines Krankheitserregers in der Probe, wobei zumindest ein Teil der Probe von einer Person stammt, die sich in dem zugangsbeschränkten Bereich befindet oder in den zugangsbeschränkten Bereich möchte,
eine Eingabeschnittstelle zum Eingeben eines Testergebnisses, das durch die Testeinrichtung mittels Testens der Probe gewonnen worden ist,
eine Probendatenbank, die dazu ausgebildet ist, eine Probe einer Identifikationskennung und/oder einer Person zuzuordnen,
eine Freigabedatenbank, die dazu ausgebildet ist, einer Identifikationskennung und/oder einer Person eine Freigabeberechtigung zuzuordnen,
ein Freigabeverwaltungssystem, das dazu ausgebildet ist, das in die Eingabeschnittstelle eingegebene Testergebnis mit einem Sollergebnis zu vergleichen und bei Erhalt eines gewünschten Vergleichsergebnisses unter Nutzung der Probedatenbank eine Freigabeberechtigung in der Freigabedatenbank zu setzen,
ein Lesegerät zum Gewinnen einer ausgelesenen Identifikationskennung durch Auslesen einer in einem Identifikationsmittel codierten Identifikationskennung,
eine Abfrageeinrichtung, die dazu ausgebildet ist, basierend auf der ausgelesenen Identifikationskennung eine vorhandene oder nicht vorhandene Freigabeberechtigung aus der Freigabedatenbank auszulesen, und
eine Signalisierungseinrichtung, die kommunizierend mit der Abfrageeinrichtung verbunden ist und die dazu ausgebildet ist, bei einer vorhandenen Freigabeberechtigung eine Freigabe des zugangsbeschränkten Bereichs zu signalisieren.

In einer Weiterbildung wird bei entsprechend kostenintensiver Bearbeitung von Einzelproben der Einsatz von geeigneten präanalytische sowie analytische Verfahren, durch Analyse von Sammelproben oder Mehrfachanalysen der gleichen Probe auf verschiedene Erreger die Effizienz erhöht.

In verfahrensmäßiger Hinsicht ist die voranstehende Aufgabe durch die Merkmale des Anspruchs 8 gelöst. Danach umfasst das in Rede stehende Verfahren die Schritte:
Nehmen einer Probe von einer Person, die sich in dem zugangsbeschränkten Bereich befindet oder in den zugangsbeschränkten Bereich möchte,
Testen der Probe auf das Vorhandensein mindestens eines Krankheitserregers in der Probe zum Gewinnen eines Testergebnisses,
Setzen einer Freigabeberechtigung in einer Freigabedatenbank, wenn ein Vergleich des Testergebnisses mit einem Sollergebnis ein gewünschtes Vergleichsergebnis liefert,
Auslesen einer Identifikationskennung mittels eines Lesegeräts zum Gewinnen einer ausgelesenen Identifikationskennung, wobei die ausgelesene Identifikationskennung in einem Identifikationsmittel codiert ist,
Auslesen einer Freigabeberechtigung aus der Freigabedatenbank für die ausgelesene Identifikationskennung und
Signalisierung einer Freigabe des zugangsbeschränkten Bereichs mittels einer Signalisierungseinrichtung bei Vorhandensein einer Freigabeberechtigung für die ausgelesene Identifikationskennung.

Es ist zunächst erkannt worden, dass ein bevölkerungsweites Testen auf eine Infektion nicht sinnvoll ist. Selbst wenn ausreichend Laborkapazitäten und Reagenzien zur Verfügung stehen, entstünde durch diese Herangehensweise ein erheblicher und nicht vertretbarer Aufwand. Erfindungsgemäß ist erkannt worden, dass es aber Bereiche gibt, für die ein Testen sinnvoll ist, nämlich Bereiche, die ohnehin zugangsbeschränkt sind oder mit geringem Aufwand in zugangsbeschränkte Bereiche umgewandelt werden können. Zu diesen Bereichen können beispielsweise Bereiche gehören, an denen durch das Zusammentreffen einer hohen Personenzahl ein erhöhtes Risiko besteht (beispielsweise Flughäfen, Skigebiete, Fußballstadien, Arbeitsstätten - sofern ein Abstandhalten technisch nicht ausreichend machbar ist), oder Orte, an denen Gesunde auf Personen mit sehr hohem Risiko bei einer Ansteckung treffen (beispielsweise Altersheime, Pflegeheime, Krankenhäuser, Arztpraxen). In diesen Bereichen kann durch das erfindungsgemäße System und das erfindungsgemäße Verfahren eine deutliche Verbesserung des Infektionsschutzes erreicht werden.

Das erfindungsgemäße System weist hierzu eine Testeinrichtung, eine Eingabeschnittstelle zum Eingeben eines Testergebnisses, eine Probendatenbank, eine Freigabedatenbank, ein Freigabeverwaltungssystem, ein Lesegerät, eine Abfrageeinrichtung sowie eine Signalisierungseinrichtung auf. Die Testeinrichtung ist dazu ausgebildet, eine Probe auf das Vorhandensein mindestens eines Krankheitserregers in der Probe zu testen. Dieser Krankheitserreger kann ein Virus sein, beispielsweise das SARS-CoV-2. Die getestete Probe stammt zumindest zu einem Teil von einer Person, die sich in dem zugangsbeschränkten Bereich befindet oder in den zugangsbeschränkten Bereich möchte. Die Eingabeschnittstelle wird dazu genutzt, ein durch die Testeinrichtung durch Testen der Probe gewonnenes Testergebnis einzugeben. Die Probedatenbank ist dazu ausgebildet, eine Probe einer Identifikationskennung und/ oder einer Person zuzuordnen. Die Freigabedatenbank ist dazu ausgebildet, einer Identifikationskennung und/oder einer Person eine Freigabeberechtigung zuzuordnen. Das Freigabeverwaltungssystem ist dazu ausgebildet, Freigabeberechtigungen in der Freigabedatenbank zu verwalten, insbesondere zu setzen und zu entziehen. Hierzu wird ein in die Schnittstelle eingegebenes Testergebnis mit einem Sollergebnis verglichen. Bei Erhalt eines gewünschtes Vergleichsergebnisses trägt das Freigabeverwaltungssystem eine Freigabeberechtigung in die Freigabedatenbank ein. Hierbei greift das Freigabeverwaltungssystem auf die Probendatenbank zu, insbesondere um eine Zuordnung der Probe zu einer Identifikationskennung und/oder einer Person zu ermitteln. Mit diesen Bestandteilen des Systems kann erreicht werden, dass eine Probe bewertet und bei einem gewünschten Ergebnis eine Freigabe erteilt wird.

Diese erteilte Freigabe kann bei einer späteren Freigabe eines zugangsbeschränkten Bereichs genutzt werden. Hierzu weist das System ein Lesegerät auf, das zum Gewinnen einer ausgelesenen Identifikationskennung durch Auslesen einer in einem Identifikationsmittel codierten Identifikationskennung ausgebildet ist. Dieses Identifikationsmittel dient zur Identifikation der Person, von der zumindest ein Teil der Probe genommen worden ist. Ein derartige Identifikationsmittel kann durch ein Armband mit Strichcode, QR (Quick Response)-Code oder RFID (Radio Frequency Identification)-Chip, eine Chipkarte (beispielsweise ein Personalausweis mit RFID-Chip), eine Magnetstreifenkarte, eine NFC (Near Field Communication)-Kennung (beispielsweise aus einem Smartphone oder einer Smartwatch) und/oder dergleichen gebildet sein. Dabei sollte gewährleistet sein, dass das Identifikationsmittel eindeutig einer Person zugeordnet werden kann. Dies kann beispielsweise dadurch erreicht werden, dass das Identifikationsmittel nicht zerstörungsfrei von einer Person zu einer anderen übergeben werden kann, beispielsweise durch eine Sollbruchstelle eines Armbands. Denkbar ist auch, dass ein biometrisches Merkmal bei der Identifizierung der Person genutzt wird, beispielsweise eine Gesichtserkennung oder einen Fingerabdruck. Dies ist insbesondere bei der Nutzung eines Smartphones mit zugehöriger App vorteilhaft einsetzbar.

Unabhängig von der Ausgestaltung des Identifikationsmittels übermittelt das Lesegerät eine ausgelesene Identifikationskennung an eine Abfrageeinrichtung, die abfragt, ob für die ausgelesene Identifikationskennung eine Freigabeberechtigung in der Freigabedatenbank eingetragen ist. Wenn eine Freigabeberechtigung vorhanden ist, wird durch die Signalisierungseinrichtung eine Freigabe des zugangsbeschränkten Bereichs signalisiert. Wenn keine Freigabeberechtigung vorhanden ist, wird die Freigabe verwehrt.

Die Datenbanken, insbesondere die Probendatenbank und die Freigabedatenbank, können auf verschiedenste Weise implementiert sein. Wichtig ist, dass die Datenbank die jeweils geforderte Zuordnung gewährleisten kann. Ob diese Zuordnung in Form einer einfachen Tabelle oder einem komplexen Datenbanksystem erfolgt, ist für die vorliegende Erfindung zweitrangig.

Auch die Proben können auf verschiedene Weise gewonnen werden, bevorzugt aus dem Nasenrachenraum. Hierbei kann die Probe mittels einer Rachenspülung gewonnen werden, die direkt in ein entsprechend großes Probenröhrchen oder beispielsweise mittels eines Strohhalms in ein kleines Probenröhrchen abgegeben wird. Die Probe kann aber auch durch Auswischen der Mund-Rachenhöhle mit einem Tupferstäbchen oder durch eine Nasenspüllösung (Nasentropfen mit anschießendem Ausspucken oder Auslassen der Nasenspüllösung über Strohhalme) genommen werden. Geeignete Verfahren sind aus der Praxis hinlänglich bekannt. Eine Probe kann für längeren Transport mit einer virusstabilisierenden Lösung versehen werden, um auch bei einem Transport von dem Ort der Probenahme zu der Testeinrichtung die Qualität der Probe nicht zu beeinträchtigen. Eine Probe sollte dabei identifizierbar sein. Dies kann dadurch erreicht werden, dass ein Probenbehältnis (beispielsweise ein Probenröhrchen) eine Probenkennung trägt, beispielsweise in Form eines Strichcodes oder eine QR-Codes, der an der Außenseite des Probenbehältnisses angebracht ist.

Das erfindungsgemäße System ist dann besonders gut nutzbar, wenn die Testeinrichtung zügig und kostengünstig eine große Anzahl von Testergebnissen liefert. Dies kann auf verschiedene Weise erreicht werden. So können auch Schnelltests genutzt werden. Beispielsweise sind für die Identifikation von Personen, welche SARS-CoV-2 ausscheiden, Testeinrichtungen am Markt verfügbar, die binnen 30 Minuten ein Testergebnis liefern. In einer Ausgestaltung kommt eine Analyse mittels klassischer PCR (Polymerase Chain Reaction) zum Einsatz oder mittels Multiplex-PCR, sollen verschiedene Erreger simultan erfasst werden. Dies kann von Bedeutung sein, wenn beispielsweise parallel zu einer zweiten Welle einer SARS-CoV-2 Ausbreitung auch andere potenziell gefährdende Erreger wie Influenza zirkulieren, die unterschiedliche Maßnahmen der öffentlichen Gesundheitsfürsorge und Bevölkerungsepidemie erfordern, beide aber im Anfangsstadium von ähnlichen klinischen Krankheitszeichen begleitet sind.

Da diese Tests in der Regel in der Einzelanalyse meist teuer sind, wird in einer Weiterbildung des Verfahrens ein "Pooling" durchgeführt. Hierbei wird mittels einer Aliquotiereinrichtung jeweils ein Aliquot aus mehreren Einzelproben gezogen, wobei ein Aliquot meist im Bereich von wenigen Milliliter oder darunter misst. Die auf diese Weise gewonnenen Aliquots werden durch eine Mischeinrichtung gemischt und damit eine Mischung mehrerer Aliquots erzeugt. Diese Mischung wird der Testeinrichtung als Probe zugeführt, die eine Testung der Mischung durchführt. Diese Herangehensweise beruht zum einen auf der Tatsache, dass einige Testverfahren - beispielsweise das PCR Verfahren - derart empfindlich ist, dass selbst geringe Mengen eines Virus sicher erkannt werden. Zum andern beruht diese Herangehensweise auf der Annahme, dass vielfach ein negativer Befund zu erwarten ist. Wenn in der Mischung der mehreren Aliquots kein Virus nachgewiesen werden kann, können sämtliche Ausgangsproben als virusfrei betrachtet werden. Wenn die Mischung einen positiven oder unbestimmten Befund liefert, müssen alle Einzelproben, aus denen Aliquots gezogen worden sind, nachgetestet werden. Da die Aliquots jeweils lediglich einen Teil der Einzelproben bilden, ist dieses Nachtesten ohne weiteres möglich. Trotz eines eventuellen Nachtestens wird auf diese Weise der Aufwand erheblich reduziert. Wenn beispielsweise 10.000 Einzelproben untersucht werden müssen und eine PCR-Analyse angewandt wird, reduziert sich bei einer Aliquotierung von 1:32 (d.h. 32 Aliquots werden gemischt) der Testaufwand an PCR-Analysen auf 313 Proben, wenn alle Proben negativ sind, auf 345 Proben, wenn eine der 10.000 Einzelproben das Virus enthält, auf 377 Proben bei 2 positiven Einzelproben, usw. Es ist zu erkennen, dass ein Pooling den Testaufwand erheblich reduziert.

Je größer die Anzahl von gemischten Aliquots ist, desto mehr Testaufwand kann eingespart werden. Wenn allerdings "zu viele" Aliquots gemischt werden, kann eine virenbelastete Einzelprobe möglicherweise nicht mehr zuverlässig genug erkannt werden. In einer Ausgestaltung besteht die Mischung der mehreren Aliquots aus mehr als 10 Aliquots. In einer anderen Ausgestaltung besteht die Mischung der mehreren Aliquots aus mehr als 20 Aliquots. In einer noch anderen Ausgestaltung werden bis zu 100 Aliquots in der Mischung der mehreren Aliquots gemischt. In einer weiteren Ausgestaltung besteht die Mischung der mehreren Aliquots aus 32 Aliquots.

Eine Aliquotierung im Zusammenhang mit dem erfindungsgemäßen System bedeutet, dass eine zu untersuchende Probe Teile mehrerer Einzelproben enthält.

Wenn in einer Mischung der mehreren Aliquots kein getesteter Krankheitserreger aufgefunden worden ist, kann - unter der Voraussetzung keiner "zu großen" Anzahl von Aliquots in der Mischung - davon ausgegangen werden, dass keine der Einzelproben mit dem Krankheitserreger belastet ist. Insofern kann ein Vergleich des Testergebnisses für die Mischung mit einem Sollergebnis als repräsentativ für alle beigemischten Einzelproben angesehen werden. Daher ist in einer Weiterbildung das Freigabeverwaltungssystem dazu ausgebildet, das Testergebnis für eine Mischung mehrerer Aliquots mit einem Sollergebnis zu vergleichen. Wenn der Vergleich ein gewünschtes Vergleichsergebnis liefert, beispielsweise "kein Befund", kann das Freigabeverwaltungssystem für alle Einzelproben eine Freigabeberechtigung setzen. Hierzu kann das Freigabeverwaltungssystem wiederum auf die Probendatenbank zugreifen. Wenn der Vergleich nicht zu einem gewünschten Vergleichsergebnis führt, kann - beispielsweise durch das Freigabeverwaltungssystem - ein Test für alle Einzelproben initiiert werden.

In einer Ausgestaltung weist die Testeinrichtung eine Ausgabeeinrichtung auf, mit der ein Testergebnis ausgegeben werden kann. Wie eine derartige Ausgabeeinrichtung konkret ausgestaltet ist, hängt von der jeweiligen Testeinrichtung ab. In einer Ausgestaltung ist die Ausgabeeinrichtung ein Sichtfenster, eine Signalleuchte oder ein Display, das/die ein Testergebnis optisch darstellt. In diesem Fall dürfte das Testergebnis manuell in die Eingabeschnittstelle eingegeben werden. In einer anderen Ausgestaltung der Ausgabeeinrichtung ist diese durch eine Ausgabeschnittstelle gebildet, die ein Testergebnis als analoges oder digitales Signal ausgeben kann. In diesem Fall kann die Eingabeschnittstelle als korrespondierende Schnittstelle ausgebildet sein, in die ein durch die Ausgabeeinrichtung ausgegebenes Testergebnis eingegeben werden kann.

Entsprechend kann die Eingabeschnittstelle verschiedentlich ausgebildet sein. Wesentlich ist lediglich, dass über die Eingabeschnittstelle eine Information eingegeben werden kann, die als Testergebnis interpretierbar ist. In einer Ausgestaltung kann die Eingabeschnittstelle eine Tastatur umfassen, in die ein Testergebnis eingegeben wird. In einer anderen Ausgestaltung kann die Eingabeschnittstelle durch ein Feld einer Bildschirmanzeige gebildet sein, das durch einen Mausklick oder ein Antippen an einem berührungsempfindlichen Display ausgewählt werden kann. In einer noch weiteren Ausgestaltung kann die Eingabeschnittstelle durch eine Schnittstelle gebildet sein, in die elektrische oder optische Signale eingegeben werden, wobei mit den Signalen ein jeweils einzugebendes Testergebnis codiert ist.

Auch die Signalisierungseinrichtung kann verschiedentlich ausgebildet sein. Hierbei ist wichtig, dass die Signalisierungseinrichtung in der Lage ist, eine Freigabe zu signalisieren. Wie dies konkret geschieht, ist zweitranging. In einer Ausgestaltung ist die Signalisierungseinrichtung für eine optische Signalisierung ausgebildet. Dies kann beispielsweise durch ein Display oder eine Leuchte geschehen, das/die eine Freigabe anzeigt. In einer Ausgestaltung ist die Signalisierungseinrichtung für eine akustische Signalisierung ausgebildet. Hierbei kann eine Freigabe durch ein erstes akustisches Signal und eine nicht erfolgte Freigabe durch ein zweites akustisches Signal erfolgen, wobei das erste akustische Signal und das zweite akustische Signal eindeutig voneinander unterscheidbar sein sollten. In einer Ausgestaltung ist die Signalisierungseinrichtung für eine mechanische Signalisierung ausgebildet. Dies kann beispielsweise dadurch erfolgen, dass ein Drehkreuz freigeschaltet oder eine Schiebetüre oder eine Schleuse geöffnet wird. Die Signalisierungseinrichtung kann auch eine optische und/oder eine akustische und/oder eine mechanische Signalisierung kombinieren.

Bei sehr vielen Krankheitserregern ist eine Ansteckungsgefahr für andere unmittelbar nach einer Infektion gering oder praktisch nicht vorhanden. Erst wenn sich der Krankheitserreger in einer infizierten Person ausreichend vermehren konnte, steigt das Ansteckungsrisiko für andere Personen. Daher kann das Freigabeverwaltungssystem in einer Weiterbildung dazu ausgebildet sein, eine in der Freigabedatenbank gesetzte Freigabeberechtigung wieder zu entziehen. Dieses Entziehen einer Freigabeberechtigung kann nach Ablauf einer vorgegebenen Zeitspanne, beispielsweise 24 Stunden oder 2 Tage, und/oder nach Erreichen eines vorgegebenen Zeitpunkts, beispielsweise Mitternacht oder 3 Uhr nachts, erfolgen. Auf diese Weise kann erreicht werden, dass eine einmal erteilte Freigabeberechtigung lediglich solange aktiv bleibt, wie ein Infektionsschutz mit hoher Wahrscheinlichkeit gewährleistet ist.

In einer Ausgestaltung wird das Nehmen einer Probe, das Testen der Probe und das Setzen einer Freigabeberechtigung regelmäßig wiederholt. Auf diese Weise kann gewährleistet werden, dass eine eventuell erfolgte Infektion einer Person nicht zu einer Reduktion des Infektionsschutzes führt.

In einer Weiterbildung ist das erfindungsgemäße System als verteiltes System ausgebildet, wobei die Bestandteile des Systems über ein Netzwerk miteinander interagieren können. Je nach Ausmaß der Verteilung der Bestandteile kann das Netzwerk durch ein lokales Netzwerk gebildet sein, beispielsweise Ethernet, WiFi oder Bluetooth, um lediglich einige mögliche Ausgestaltungen zu nennen. Insbesondere wenn sich Bestandteile in größeren Entfernungen zueinander befinden, beispielsweise mehrere Kilometer entfernt, bietet sich die Nutzung des Internets an, wobei die Kommunikation verschlüsselt erfolgen sollte. Entsprechende Verschlüsselungstechniken (beispielsweise SSL - Secure Socket Layer) und Zugangssysteme (beispielsweise DSL - Digital Subscriber Line, LTE - Long Term Evolution oder GSM - Global System for Mobile Communication) sind aus der Praxis hinlänglich bekannt. Durch Nutzung eines verteilten Systems kann eine Probenahme an einem ersten Ort, ein Testen der Probe an einem zweiten Ort, eine Freigabeverwaltung an einem dritten Ort und eine Nutzung der Freigabeberechtigung an einem vierten Ort erfolgen. Dies ermöglicht auch, dass der zugangsberechtigte Bereich nicht im selben Gebäude wie der Ort der Probenahme liegen muss. So kann der zugangsberechtigte Bereich in derselben Stadt wie der Ort der Probenahme, in derselben Region oder demselben Land liegen. Insbesondere bei der Nutzung des Systems bei Flugreisen kann der zugangsberechtigte Bereich aber auch in einem anderen Land oder auf einem anderen Kontinent liegen, um beispielsweise ein Einschleppen des getesteten Krankheitserregers in das Zielland zu vermeiden.

In einer Weiterbildung umfasst das System eine Identifikationsdatenbank, die einer Person ein Identifikationsmittel und/oder eine in dem Identifikationsmittel codierte Identifikationskennung zuordnet. Die entsprechende Zuordnung kann bei Ausgabe des Identifikationsmittels an die betreffende Person gespeichert werden. Es ist aber auch denkbar, dass die Zuordnung im Rahmen eines Registrierungsprozesses erfolgt, beispielsweise im Rahmen der Aktivierung einer App auf einem Smartphone oder einem Tablet.

In einer Ausgestaltung des erfindungsgemäßen Verfahrens kann vor oder bei Betreten des zugangsbeschränkten Bereichs ein in einem Identifikationsmittel codierte Identifikationskennung ausgelesen werden. Mit der derart ausgelesenen Identifikationskennung wird dann in der Freigabedatenbank angefragt, ob eine Freigabeberechtigung für die ausgelesene Identifikationskennung vorliegt. Wenn eine Freigabeberechtigung vorliegt, wird der Zutritt zu dem zugangsbeschränkten Bereich freigegeben. Diese Herangehensweise bietet sich überall dort an, wo das Tragen einer Mund-Nase-Maske oder das Einhalten eines Mindestabstands nicht möglich ist oder besonderer Infektionsschutz erforderlich ist. Beispielhaft sei auf die Anwendung bei einem Wellnessbereich, einer Pflegestation, einem Altenheim oder einem Fußballstadion genannt.

In einer Ausgestaltung des erfindungsgemäßen Verfahrens kann eine in einem Identifikationsmittel codierte Identifikationskennung bei Verlassen des zugangsbeschränkten Bereichs ausgelesen werden. Für die derart ausgelesene Identifikationskennung wird eine Freigabeberechtigung aus der Freigabedatenbank abgefragt. Wenn eine Freigabeberechtigung vorhanden ist, darf der zugangsbeschränkte Bereich in eine gewünschte Richtung verlassen werden. Diese Herangehensweise bietet sich insbesondere bei Transitbereichen an, bei denen innerhalb des Transitbereichs durch Mund-Nase-Masken und Abstandregelungen ein Infektionsschutz erreicht werden kann. Wenn der Transitbereich in Transitrichtung verlassen werden soll, beispielsweise zum Besteigen eines Flugzeuges, wird die Freigabeberechtigung geprüft. In dem Bereich hinter dem Transitbereich ist damit sichergestellt, dass das Infektionsrisiko für den getesteten Krankheitserreger nahezu Null ist.

In einer Weiterbildung kann ein Betreten und/oder ein Verlassen des zugangsbeschränkten Bereichs in einer Protokolldatenbank erfasst werden. Dabei kann ein Zeitstempel und/oder eine ausgelesene Identifikationskennung protokolliert werden. Auf diese Weise ist nachvollziehbar, welche Personen sich gleichzeitig in dem zugangsbeschränkten Bereich aufgehalten haben.

In einer Weiterbildung des erfindungsgemäßen Verfahrens können Quarantänemaßnahmen ausgelöst werden, wenn ein Vergleich des Testergebnisses mit einem Sollergebnis ein undefiniertes oder unerwünschtes Vergleichsergebnis liefert. Dies kann immer dann angebracht sein, wenn das Testergebnis das Vorliegen einer Infektion mit dem getesteten Krankheitserreger nahelegt oder eine Infektion zumindest nicht ausreichend sicher ausgeschlossen werden kann.

Es gibt nun verschiedene Möglichkeiten, die Lehre der vorliegenden Erfindung in vorteilhafter Weise auszugestalten und weiterzubilden. Dazu ist einerseits auf die den nebengeordneten Ansprüchen nachgeordneten Ansprüche und andererseits auf die nachfolgende Erläuterung bevorzugter Ausführungsbeispiele der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: ein Anwendungsszenarium eines Ausführungsbeispiels eines erfindungsgemäßen Systems bzw. eines erfindungsgemäßen Verfahrens im Zusammenhang mit einem Skigebiet,
- Fig. 2: ein Anwendungsszenarium eines Ausführungsbeispiels eines erfindungsgemäßen Systems bzw. eines erfindungsgemäßen Verfahrens im Zusammenhang mit einer Flugreise und
- Fig. 3: ein Anwendungsszenarium eines Ausführungsbeispiels eines erfindungsgemäßen Systems bzw. eines erfindungsgemäßen Verfahrens im Zusammenhang mit einem Wellness-Hotel.

Die Erfindung ermöglicht ein kostengünstiges und praktikables Verfahren, wie durch gezielte Testung von Rachenspül-Lösung oder auf andere Weise gewonnene Proben aus dem Nasenrachenraum an Orten mit prinzipiell möglicher Zugangskontrolle in Zeiten von epidemischer Verbreitung einer Infektion dennoch Sicherheit für die Besucher und Beschäftigten erreicht werden kann. Das Prinzip beruht auf dem Umstand, dass Personen etwa mit SARS-CoV-2 Viruserkrankung kurz vor dem Ausbruch der Symptome besonders ansteckend sind. Besonders ansteckend zu sein bedeutet, dass die Schleimhäute des Nasenrachenraumes in hoher Anzahl Viren freisetzen und mit Atmung oder Husten aber auch über den Speichel verbreiten.

Mittels Gurgeln einer geeigneten Spüllösung werden wenige Milliliter Probe von jeder Person beim Einlass oder früher an dem gleichen Tag gewonnen. Die Identität des Probengebers wird durch entsprechende sichere Verfahren mit dem Barcode-markierten Probenröhrchen durch Scanner fehlersicher für eine spätere Nachverfolgung verbunden.

Die Proben werden in einer virusstabilisierenden Lösung zeitnah in ein Analyselabor verbracht. Dort wird aus mehreren Proben (gesichert nach aktuellem Wissensstand bis 32 Proben) ein Aliquot gezogen und vermischt mit den anderen Aliquots in einer PCR-Reaktion auf das gesuchte Virus (beispielsweise SARS-CoV-2) untersucht. Zeigt die Mischprobe keine Anwesenheit des Virus, so gelten alle Ausgangsproben als unauffällig. Zeigt die Mischprobe Anwesenheit von Virus, so müssen alle Ursprungsproben einzeln nachuntersucht werden. Da eine PCR Reaktion auf klassischen Analyseautomaten wenige Stunden erfordert, können bis zum Ende des Arbeitstages auf diese Weise alle potenziell positiven Proben identifiziert werden.

Sollte sich eine positive Person finden, kann diese unmittelbar am gleichen Abend informiert werden und sie und alle anderen Kontaktpersonen in Quarantäne versetzt werden. Da die Kontaktpersonen frühestens einige Tage nach dem Kontakt mit der infizierten und den Erreger verbreitenden Person selbst ansteckend werden, ist dadurch die Ausbreitung zweifelsfrei unterbrochen. Sollte am Tag nach der Einlasskontrolle eine der untersuchten Personen Krankheitszeichen entwickeln, ist erstens sichergestellt, dass diese Personen mit äußerst hoher Wahrscheinlichkeit keine SARS-CoV-2 Infektion hat, sondern dass die Erkrankung von einem anderen Erreger verursacht sein muss.

Das Verfahren sieht optional vor, dass die zurückbehaltene Probe vom Vortag, dem Zeitpunkt der größten Ansteckung, mittels Multiplex-PCR (d.h. gleichzeitige Untersuchung auf verschiedene Erreger) und anschließender genomischer Next-Generation-Sequenzierung untersucht wird, um so positiv die mutmaßliche Ursache der Erkrankung zu bestimmen. Durch diese Option trägt das Verfahren weiter zur Sicherheit bei durch die Unterscheidung zwischen dem eher seltenen zu erwartenden Ereignis einer SARS-CoV-2 Infektion und den sehr viel häufiger zu erwartenden Infektionen mit anderen Erregern von Atemwegserkrankungen und grippalen Infekten bis hin zur Influenza.

Das Verfahren eignet sich also vor allem, wenn die Ausgangswahrscheinlichkeit des gesuchten gefährlichen Erregers, wie in der aktuellen Situation in Europa mit SARS-CoV-2, eher sehr gering ist, gleichwohl aufgrund der von einer unerkannten Infektionsausbreitung ausgehenden Gefahr für die Bevölkerung einschneidende Schutzmaßnahmen erforderlich sind. Das Verfahren schafft hier Sicherheit beim Zugang, kann in Kombination mit anderen Zugangskontrollen wie Scannern, RFID-Lesern in unterschiedlichsten Situationen eingesetzt werden, angefangen von dem Zutritt zu Wellnessbereichen in Hotels, Zutritt zu Gaststätten, Zutritt zu Veranstaltungen, Zutritt zu Sportanlagen, einschließlich Skigebieten, Zutritt zu Arbeitsstätten und Produktionsanlagen. Insbesondere bei Nutzung einer Aliquotierung reduziert das Verfahren gegenüber den heute üblichen Testverfahren massiv die Belastung für die Untersuchten und die Kosten.

Das erfindungsgemäße Verfahren behebt diverse Schwachstellen der jetzigen Routinen, es erhöht die Reaktionszeit bei Ausbrüchen, es vermindert die Kosten, es erhöht die Sicherheit, es erhöht die Beruhigung und vor allem erhöht es die Möglichkeit, trotz weiterem Vorhandensein des Virus in der Bevölkerung Anlässe mit Zusammentreffen vieler Personen zu ermöglichen, in denen Social Distancing nur erschwert möglich ist, zum Beispiel Reisen im Großraumflugzeug oder Konzerte. Es ist anwendbar überall dort, wo eine Zugangskontrolle ohnehin vorgesehen ist oder leicht eingerichtet werden kann. Überall dort, wo andere Systeme das Tracking vollziehen, beispielsweise die Keycards beim Skigebiet, können diese Systeme genutzt werden, um bei einem positiven Befund die Zahl der möglichen Kontaktpersonen weiter einzugrenzen. Das Verfahren kann bei sehr niedriger Infektionsrate adaptiert werden, indem beispielsweise nur jede zweite oder dritte Person getestet wird (z. B. nach Geburtsdatum).

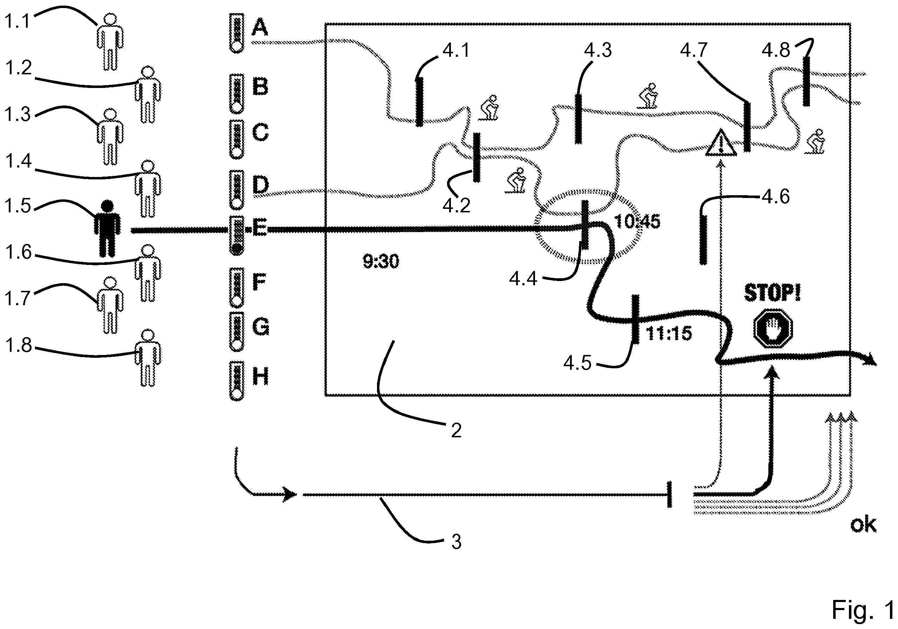

Ein möglicher Anwendungsfall ist ein Skigebiet, ein Stadion oder dergleichen. Ein derartiger Anwendungsfall ist in Fig. 1 skizziert. Es sind verschiedene Lesegeräte vorhanden, die beispielsweise ein RFID-Gating ermöglichen.

In dem dargestellten Beispiel möchten mehrere Personen 1.1 bis 1.8 einen zugangsbeschränkten Bereich 2 - hier ein Skigebiet - betreten. Vor Betreten des Bereichs 2 gibt jede Person 1.1 bis 1.8 eine Probe A bis H, wobei eine Zuordnung zwischen Person und Probe bzw. zwischen einer die Person kennzeichnenden Identifikationskennung und einer Probe jeweils in einer Probendatenbank gespeichert wird. Die Proben werden durch eine Testeinrichtung auf das Vorhandensein eines Krankheitserregers getestet, was symbolisch durch eine Zeitlinie 3 für eine Laborverarbeitung dargestellt ist. Währenddessen halten sich die Personen 1.1 bis 1.8 in dem Skigebiet auf. Der Übersichtlichkeit wegen sind lediglich die Wege von drei Personen eingezeichnet, nämlich Person 1.1 / Probe A, Person 1.4 / Probe D und Person 1.5 / Probe E. Zur Nachverfolgung der Wege der Personen sind mehrere Lesegeräte 4.1 bis 4.8 in dem Bereich 2 verteilt, die eine Identifikationskennung aus einem Identifikationsmittel der Personen 1.1 bis 1.8 auslesen können. Die Lesegeräte 4.1 bis 4.8 können als RFID-Gates ausgebildet sein. Die ausgelesenen Identifikationskennungen werden mit einem Zeitstempel in einer Protokolldatenbank abgespeichert. Einige Lesegeräte (4.3, 4.4, 4.7, 4.8) registrieren, dass sich mehrere Personen zur gleichen Zeit in dem gleichen Bereich aufhalten.

Am Ende der Laborverarbeitung 3 liegen Testergebnisse vor, wonach die Probe E den getesteten Krankheitserreger enthält. Über eine Probendatenbank lässt sich Probe E der Person 1.5 bzw. einem durch diese Person getragenen Identifikationsmittel zuordnen. Person 1.5 kann dann in dem Bereich 2 ausfindig gemacht und aus dem Bereich 2 geleitet werden, was mit dem Wort "STOP!" angedeutet ist. Mithilfe der Protokolldatenbank kann ausfindig gemacht werden, ob Kontaktpersonen vorhanden sind. In dem dargestellten Beispiel hat sich Person 1.4 um 10:45 Uhr in dem Bereich des Lesegeräts 4.4 befunden, die beispielsweise dieselbe Gondel einer Seilbahn kennzeichnen kann. Diese Person 1.4 kann entsprechend informiert werden.

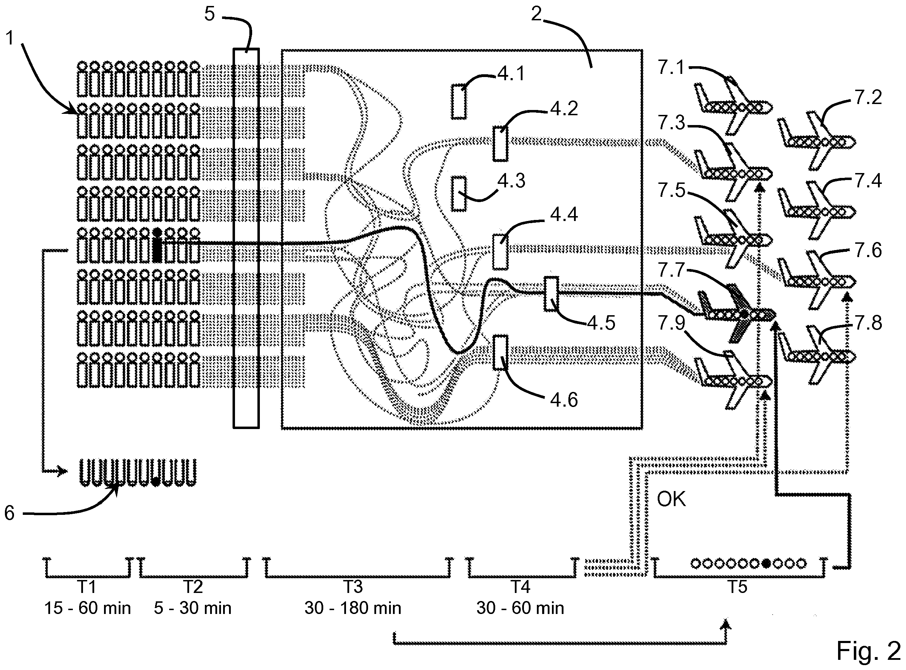

Ein anderer möglicher Anwendungsfall sind Flugreisen, wie er in Fig. 2 skizziert ist. Flugreisende 1 können bei der Sicherheitskontrolle 5 nach dem Check-In jeweils eine Probe 6 Rachen-Spüllösung abgeben müssen, bevor sie in den zugangsbeschränkten Bereich 2 - hier den Sicherheitsbereich des Flughafens - eintreten dürfen. Diese Proben 6 können in ein Labor verbracht werden und dort mittels der beschriebenen Methode analysiert werden. Im Labor kann ein Pooling durchgeführt werden.

Im unteren Bereich der Fig. 2 ist ein beispielhafter Zeitstrahl aufgezeichnet. Im Zeitraum T1, der in diesem Beispiel 15 bis 60 Minuten dauert, werden die Proben in das Labor gebracht. Während des Zeitraums T2 (hier 5 bis 30 Minuten) wird eine Voranalyse durchgeführt. Im Zeitraum T3 (hier 30 bis 180 Minuten) wird die eigentliche Testung durchgeführt, beispielsweise mittels PCR. Während des Zeitraums T4 (hier 30 bis 60 Minuten) werden die Ergebnisse validiert. Sofern eine Mischung von Aliquotes einen positiven Befund aufweist, wird in Zeitraum T5 eine Nachanalyse der Einzelproben des Pools durchgeführt. Bereits nach relativ kurzer Zeit steht damit das Ergebnis bereit und kann mit dem Buchungscode der Airline verknüpft werden. Je nach Gesamtdauer der Laborverarbeitung kann bei Betreten eines Flugzeugs 7.1 bis 7.9 oder vor/bei Erreichen des Zielflughafens ein Testergebnis vorliegen und entsprechend reagiert werden.

Innerhalb des Bereichs 2 könnte an verschiedenen Stellen die Bordkarte - ein Identifikationsmittel im Sinne der vorliegenden Erfindung - mit einem Lesegerät 4.1 bis 4.6 ausgelesen werden. Diese Lesegeräte könnten beispielsweise bei den Gates und/oder anderen Durchlässen stehen. Wenn an allen möglichen Kontaktstellen innerhalb des Flughafens die Bordkarte vorgezeigt werden müsste, könnte über die Verknüpfung der Systeme der exakte Pfad und die Kontaktpersonen der Betroffenen identifiziert werden. Damit wäre eine rasche Eindämmung sichergestellt. Für die weit überwiegende Mehrzahl der Flugreisenden würde sich der Kapitän beispielsweise auf einem Flug nach New York etwa bei Island melden und eine Freigabe für alle Fluggäste kommunizieren. In dem dargestellten Beispiel befindet sich in Flugzeug 7.7 eine Person, die positiv getestet worden ist. In den anderen Flugzeugen befindet sich keine Person, bei denen der getestete Krankheitserreger nachgewiesen werden konnte.

Wenn durch entsprechenden Fortschritt der Laborverfahren bereits 30 Minuten nach der Probenabnahme ein Testergebnis vorliegt, kann das Testergebnis bzw. die Freigabe bereits beim Boarding abgefragt werden. Hier ist denkbar, dass lediglich die Passagiere das Flugzeug 7.1 bis 7.9 betreten dürfen, die ein negatives Testergebnis haben. Auf diese Weise ist mit Abschluss des Boarding mit hoher Wahrscheinlichkeit sichergestellt, dass nur aktuell nicht infektiöse Reisende das Flugzeug betreten haben. Auf Schutzmaßnahmen während des Fluges kann dann verzichtet werden.

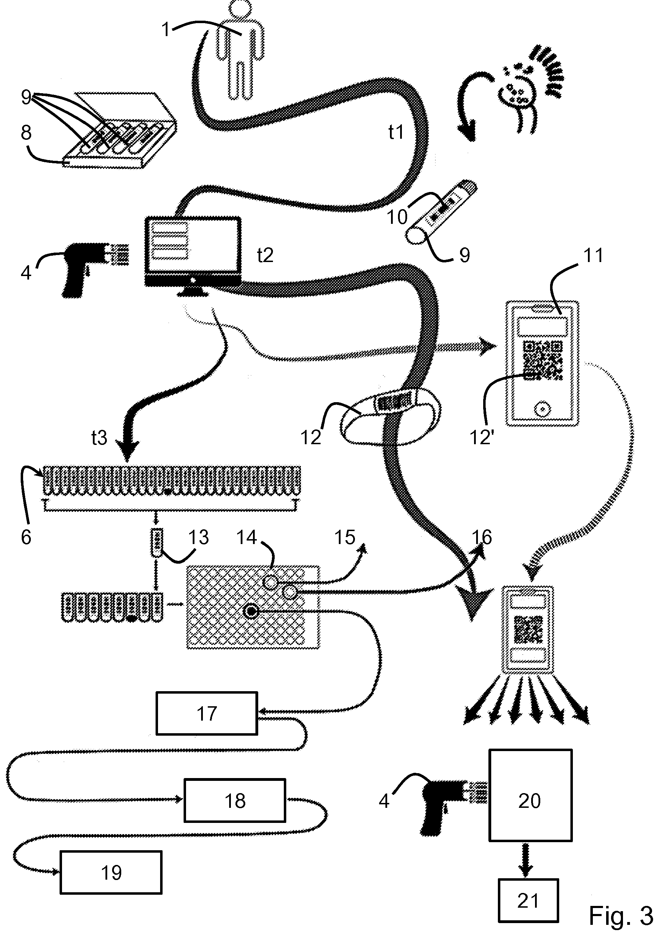

Ein anderer möglicher Anwendungsfall sind Hotelaufenthalte, wie er in Fig. 3 skizziert ist. Ein entsprechender Ablauf könnte wie folgt aussehen:
1. In dem Hotel ankommende Gäste 1 erhalten beim Check-In eine AmpullenSchachtel 8 mit Abnahmeröhrchen für mehrere Tage, Rachenspüllösung für mehrere Tage sowie Strohhalme für das Verbringen der Rachenspüllösung in ein Probenröhrchen 9.
2. Zum Zeitpunkt t1 gurgeln die Gäste 1 mit der Rachenspüllösung und verbringen die Probe in ein Probenröhrchen 9. Die Gäste 1 geben am Ankunftstag die erste Probe ab. Bei Probenabgabe nach 9:00 Uhr gelten für diese Gäste am Anreisetag die allgemeinen Schutz- und Hygienevorschriften.
3. Die Rezeption oder ein entsprechend einzurichtender Stand nimmt zum Zeitpunkt t2 unter Einhaltung der Hygienevorschriften das mit einem Barcode 10 versehene Probenröhrchen 9 entgegen und legt über eine Website den Gast anonym an. Optional kann der Gast sich auch persönlich registrieren, beispielsweise mittels eines Smartphones 11.
4. Die Rezeption scannt den Barcode des Probenröhrchen. Die Informationen werden in einer Probendatenbank abgelegt.
5. Die Rezeption scannt bei Abgabe der Probe vor 9:00 Uhr ein ebenfalls einen (anderen) Barcode tragendes, industriell vorgefertigtes Armband - Identifikationsmittel 12 - und übergibt dies dem Gast. Alternativ oder zusätzlich kann ein QR-Code auf dem Smartphone 11 ein Identifikationsmittel 12' bilden.
6. Die Rezeption trägt in der Software die Zimmernummer und die fortlaufende Nummer des Gastes im Zimmer (Gast 1, Gast 2 usw.) ein. Auf diese Weise bleibt die vollständige Anonymität der Gäste erhalten. Die Zuordnung wird in einer Identifikationsdatenbank gespeichert.
7. Auf Wunsch kann die Rezeption zusätzlich eine gültige E-Mail-Adresse des Gastes eintragen, die dann dem Gast ermöglicht sich im System anzumelden und beispielsweise frühzeitig das Testergebnis zu erfahren oder bei Auftreten von Krankheitszeichen die eigenen Symptome einzutragen. Dies erleichtert im seltenen Fall einer Erkrankung (z. B. normale Erkältung) die weitere Behandlungsführung. Gäste können zunächst auch nur anonym teilnehmen und sich jeden Tag neu für das Einrichten des Logins entscheiden. Der Vorteil des Logins ist, dass der gleiche Code wie auf dem Armband auch auf dem Smartphone angezeigt werden kann, wie eine Bordkarte zum Check-in beim Flugzeug.
8. Das Labor holt durch einen Fahrdienst nach 9:00 Uhr die Proben ab.
9. Zum Zeitpunkt t3 werden Aliquotes aus mehreren Proben 6 (beispielsweise 10 bis 30 Proben) gezogen und zu einer Mischung 13 der mehreren Proben 6 gemischt. Die Mischung 13 wird einer Testeinrichtung 14 zugeführt, die in Fig. 3 lediglich symbolisch dargestellt ist und beispielsweise ein High Throughput PCT oder eine alternative Methode nutzen kann.
10. Das Hotel erhält bis spätestens 15:00 Uhr im System automatisch die vollständige Freigabe, sofern alle Proben negativ sind (Pfeil 15). Zusätzlich kann der Gast separat informiert werden (Pfeil 16), beispielsweise über sein Smartphone 11 oder per E-Mail. In dem selten zu erwartenden Fall erhält das Hotel gegen 15:00 Uhr die Vorinformationen, dass mindestens eine Probe positiv war und daher die Schutzmaßnahmen fortgeführt werden müssen (Block 17), bis etwa gegen 19:00 Uhr die positive Einzelprobe bestätigt ist. Bei einer positiven Einzelprobe wird eine Meldung an den betroffenen Gast abgesetzt (Block 18), der beispielsweise über die Zimmernummer identifiziert werden kann. Der betreffende Gast kann unter Quarantäne gestellt werden (Block 19). Kontaktpersonen müssen nicht zwingend in Quarantäne, sollten aber Vorsicht walten lassen und täglich getestet werden. Die Freigaben werden als Freigabeberechtigungen in einer Freigabedatenbank eingetragen. Das Probennehmen, Testen, Benachrichtigen und Setzen von Freigaben kann täglich wiederholt werden.
11. Das Hotel muss ferner alle Angestellten täglich testen, besonders diejenigen, die in den offenen Bereichen tätig sind oder mit Gästen in Kontakt treten könnten.
12. Ab 15:00 Uhr kann das Hotel dann sämtliche zugangskontrollierbaren Bereiche vollständig öffnen und hier auf die Schutzmaßnahmen bis auf die Desinfektion nach Schließung der Einrichtung am Ende des Tages verzichten. Die Zugangskontrolle 20 erfolgt über Barcodescanner oder ein anderes Lesegerät 4. Gescannt wird in diesem Beispiel das Identifikationsmittel 12, 12', d.h. das Armband oder der QR-Code aus dem Smartphone 11. Wenn für eine ausgelesene Identifikationskennung in der Freigabedatenbank eine Freigabe eingetragen ist, erhält der Gast Zutritt in den zugangsbeschränkten/zugangskontrollierten Bereich (Block 21). Das System kennt alle an diesem Tag negativen Proben.

Prinzipiell ist auch denkbar, dass lediglich für neu eingecheckte Gäste ohne Freigabeberechtigung Schutzmaßnahmen gelten und dass die zugangsbeschränkten Bereiche für alle bereits getesteten Gäste ganztägig ohne Schutzmaßnahmen offen sind. Dennoch müssen diese Gäste täglich eine Probe abgeben, um die Freigabeberechtigung zu behalten.

Hinsichtlich weiterer vorteilhafter Ausgestaltungen wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele lediglich zur Erörterung der beanspruchten Lehre dienen, diese jedoch nicht auf die Ausführungsbeispiele einschränken.

### Bezugszeichenliste

- 1: Person
- 2: zugangsbeschränkter Bereich
- 3: Zeitlinie für Laborverarbeitung
- 4: Lesegeräte
- 5: Sicherheitskontrolle
- 6: Probe
- 7: Flugzeug
- 8: Ampullenschachtel
- 9: Proberöhrchen
- 10: Barcode
- 11: Smartphone
- 12: Identifikationsmittel
- 13: Mischung mehrerer Proben
- 14: Testeinrichtung
- 15: Pfeil "Hotel, alles Grün"
- 16: Pfeil: "Gast, alles Grün"
- 17: Positiver Pool, Nachtesten
- 18: Meldung an Gast
- 19: Quarantäne für Gast mit positivem Testergebnis
- 20: Zugangskontrolle
- 21: Zutritt in zugangsbeschränkten Bereich

## Patentansprüche

1. System zum Infektionsschutz in einem zugangsbeschränkten Bereich, umfassend:
eine Testeinrichtung zum Testen einer Probe auf das Vorhandensein mindestens eines Krankheitserregers in der Probe, wobei zumindest ein Teil der Probe von einer Person stammt, die sich in dem zugangsbeschränkten Bereich befindet oder in den zugangsbeschränkten Bereich möchte,
eine Eingabeschnittstelle zum Eingeben eines Testergebnisses, das durch die Testeinrichtung mittels Testens der Probe gewonnen worden ist,
eine Probendatenbank, die dazu ausgebildet ist, der Probe eine Identifikationskennung und/oder eine Person zuzuordnen,
eine Freigabedatenbank, die dazu ausgebildet ist, der Identifikationskennung und/oder der Person eine Freigabeberechtigung zuzuordnen,
ein Freigabeverwaltungssystem, das dazu ausgebildet ist, das in die Eingabeschnittstelle eingegebene Testergebnis mit einem Sollergebnis zu vergleichen und bei Erhalt eines gewünschten Vergleichsergebnisses unter Nutzung der Probedatenbank die Freigabeberechtigung in der Freigabedatenbank zu setzen,
ein Lesegerät zum Gewinnen einer ausgelesenen Identifikationskennung durch Auslesen einer in einem Identifikationsmittel der Person codierten Identifikationskennung,
eine Abfrageeinrichtung, die dazu ausgebildet ist, basierend auf der ausgelesenen Identifikationskennung eine vorhandene oder nicht vorhandene Freigabeberechtigung aus der Freigabedatenbank auszulesen, und
eine Signalisierungseinrichtung, die kommunizierend mit der Abfrageeinrichtung verbunden ist und die dazu ausgebildet ist, bei der vorhandenen Freigabeberechtigung eine Freigabe des zugangsbeschränkten Bereichs zu signalisieren.

2. System nach Anspruch 1, **dadurch gekennzeichnet, dass** das System eine Aliquotiereinrichtung und eine Mischeinrichtung umfasst, wobei die Aliquotiereinrichtung dazu ausgebildet ist, jeweils ein Aliquot aus mehreren Einzelproben zu ziehen, wobei die Mischeinrichtung dazu ausgebildet ist, die mehreren Aliquots zu einer Probe zu mischen, und wobei die Testeinrichtung einen Test der Mischung der mehreren Aliquots durchführt, wobei die Mischung der mehreren Aliquots mehr als 10 Aliquots, vorzugsweise mehr als 20 Aliquots, besonders bevorzugter Weise bis zu 100 Aliquots, ganz besonders bevorzugter Weise 32 Aliquots umfassen kann, und/oder wobei das Freigabeverwaltungssystem dazu ausgebildet ist das Testergebnis für die Mischung der mehreren Aliquots mit dem Sollergebnis zu vergleichen, und wobei das Freigabeverwaltungssystem zusätzlich dazu ausgebildet sein kann, bei Erhalt eines gewünschten Vergleichsergebnisses unter Nutzung der Probendatenbank die Freigabeberechtigung für alle Einzelproben der Mischung zu setzen.

3. System nach Anspruch 1 oder 2, **dadurch gekennzeichnet, dass** die Testeinrichtung zum Durchführen einer Analyse mittels PCR - Polymerase Chain Reaction - ausgebildet ist.

4. System nach einem der Ansprüche 1 bis 3, **dadurch gekennzeichnet, dass** die Testeinrichtung eine Ausgabeeinrichtung zum Ausgeben des Testergebnisses umfasst, wobei ein über die Ausgabeeinrichtung ausgegebenes Testergebnis vorzugsweise in die Eingabeschnittstelle zum Eingeben des Testergebnisses eingegeben wird.

5. System nach einem der Ansprüche 1 bis 4, **dadurch gekennzeichnet, dass** die Signalisierungseinrichtung zu einer optischen und/oder mechanischen und/oder akustischen Anzeige der Freigabe ausgebildet ist und/oder dass das Freigabeverwaltungssystem zusätzlich dazu ausgebildet ist, in der Freigabedatenbank gesetzte Freigabeberechtigungen nach Ablauf einer vorgegebenen Zeitspanne und/oder nach Erreichen eines vorgegebenen Zeitpunkts zu entziehen und/oder dass das System als verteiltes System ausgebildet ist, wobei Bestandteile des Systems über ein Netzwerk, vorzugsweise das Internet, miteinander interagieren können.

6. System nach einem der Ansprüche 1 bis 5, **dadurch gekennzeichnet, dass** das Lesegerät zum Auslesen des Identifikationsmittels im Form eines Strichcodes, eines QR-Codes - Quick Response Code, eines RFID-Chips - Radio Frequency Identification Chip und/oder einer NFC-Kennung - Near Field Communication Kennung - ausgebildet ist.

7. System nach einem der Ansprüche 1 bis 6, **dadurch gekennzeichnet, dass** das System eine Identifikationsdatenbank aufweist, die der Person das Identifikationsmittel und/oder die in dem Identifikationsmittel codierte Identifikationskennung zuordnet.

8. Verfahren zum Infektionsschutz in einem zugangsbeschränkten Bereich, vorzugsweise unter Nutzung eines Systems nach einem der Ansprüche 1 bis 7, umfassend die Schritte:
Nehmen einer Probe von einer Person, die sich in dem zugangsbeschränkten Bereich befindet oder in den zugangsbeschränkten Bereich möchte,
Zuordnen der Probe zu einer Identifikationskennung und/oder zu der Person in einer Probendatenbank,Testen der Probe auf das Vorhandensein mindestens eines Krankheitserregers in der Probe zum Gewinnen eines Testergebnisses,
Setzen einer Freigabeberechtigung in einer Freigabedatenbank, wenn ein Vergleich des Testergebnisses mit einem Sollergebnis ein gewünschtes Vergleichsergebnis liefert, wobei die Freigabedatenbank dazu ausgebildet ist, der Identifikationskennung und/oder der Person die Freigabeberechtigung zuzuordnen,
Auslesen einer Identifikationskennung mittels eines Lesegeräts zum Gewinnen einer ausgelesenen Identifikationskennung, wobei die ausgelesene Identifikationskennung in einem Identifikationsmittel der Person codiert ist,
Auslesen der Freigabeberechtigung aus der Freigabedatenbank für die ausgelesene Identifikationskennung und
Signalisierung einer Freigabe des zugangsbeschränkten Bereichs mittels einer Signalisierungseinrichtung bei Vorhandensein der Freigabeberechtigung für die ausgelesene Identifikationskennung, wobei das Nehmen einer Probe, das Testen der Probe und das Setzen einer Freigabeberechtigung regelmäßig, vorzugsweise täglich, durchgeführt werden kann.

9. Verfahren nach Anspruch 8, **dadurch gekennzeichnet, dass** die gesetzte Freigabeberechtigung nach Ablauf einer Zeitspanne und/oder bei Erreichen eines Zeitpunkts gelöscht wird.

10. Verfahren nach Anspruch 8 oder 9, **dadurch gekennzeichnet, dass** bei Ausgabe des Identifikationsmittels an die Person das Identifikationsmittel und/oder eine in dem Identifikationsmittel codierte Identifikationskennung zusammen mit Identifikationsdaten der Person in einer Identifikationsdatenbank gespeichert wird.

11. Verfahren nach einem der Ansprüche 8 bis 10, **dadurch gekennzeichnet, dass** einer Probe vor oder nach dem Gewinnen der Probe eine Probenkennung zugeordnet wird und dass die Probenkennung in einer Probendatenbank der Identifikationskennung und/oder der Person zugeordnet wird.

12. Verfahren nach einem der Ansprüche 8 bis 11, **dadurch gekennzeichnet, dass** vor oder bei Betreten des zugangsbeschränkten Bereichs mittels des Lesegeräts eine in dem Identifikationsmittel codierte Identifikationskennung ausgelesen wird, dass für die ausgelesene Identifikationskennung die Freigabeberechtigung aus der Freigabedatenbank abgefragt wird und dass bei Vorhandensein der Freigabeberechtigung ein Zutritt zu dem zugangsbeschränkten Bereich gewährt wird.

13. Verfahren nach einem der Ansprüche 8 bis 12, **dadurch gekennzeichnet, dass** der zugangsbeschränkte Bereich einen Transitbereich umfasst, dass bei Verlassen des zugangsbeschränkten Bereichs die in dem Identifikationsmittel codierte Identifikationskennung ausgelesen wird, dass für die ausgelesene Identifikationskennung die Freigabeberechtigung aus der Freigabedatenbank abgefragt wird und dass der zugangsbeschränkte Bereich erst dann in eine Transitrichtung verlassen werden kann, wenn die Freigabeberechtigung vorhanden ist.

14. Verfahren nach einem der Ansprüche 8 bis 13, **dadurch gekennzeichnet, dass** ein Betreten und/oder ein Verlassen des zugangsbeschränkten Bereichs in einer Protokolldatenbank, vorzugsweise jeweils zusammen mit einem Zeitstempel und/oder der ausgelesenen Identifikationskennung, erfasst wird.

15. Verfahren nach einem der Ansprüche 8 bis 14, **dadurch gekennzeichnet, dass** Quarantänemaßnahmen ausgelöst werden, wenn der Vergleich des Testergebnisses mit dem Sollergebnis ein undefiniertes oder unerwünschtes Vergleichsergebnis liefert.

## Claims

1. System for infection protection in a restricted-access area, comprising:
a test device for testing a sample for the presence of at least one pathogen in the sample, wherein at least a portion of the sample originates from a person who is located in the restricted-access area or who wishes to enter the restricted-access area,
an input interface for inputting a test result which has been obtained by the test device by testing the sample,
a sample database which is configured to associate an identification marker and/or a person with the sample,
a release database which is configured to associate a release authorisation with the identification marker and/or the person,
a release management system which is configured to compare the test result input into the input interface with a desired result and, when a desired comparison result is obtained using the sample database, to set the release authorisation in the release database,
a reading device for obtaining an output identification marker by outputting an identification marker which is encoded in an identification means of the person,
a request device which is configured, based on the output identification marker, to output a release authorisation which may or may not be present from the release database and a signalling device which is connected in a communicating manner to the request device and which is configured, when the release authorisation is present, to signal a release of the restricted-access area.

2. System according to claim 1, **characterised in that** the system comprises an aliquoting device and a mixing device, wherein the aliquoting device is configured to in each case draw an aliquot from a plurality of individual samples, wherein the mixing device is configured to mix the plurality of aliquots into a sample, and wherein the test device carries out a test of the mixture of the plurality of aliquots, wherein the mixture of the plurality of aliquots may comprise more than 10 aliquots, preferably more than 20 aliquots, in a particularly preferred manner up to 100 aliquots, in a very particularly preferred manner 32 aliquots, and/or wherein the release management system is configured to compare the test result for the mixture of the plurality of aliquots with the desired result, and wherein the release management system may additionally be configured, when a desired comparison result is obtained using the sample database, to set the release authorisation for all the individual samples of the mixture.

3. System according to claim 1 or 2, **characterised in that** the test device is configured to carry out an analysis by means of PCR - Polymerase Chain Reaction.

4. System according to any one of claims 1 to 3, **characterised in that** the test device comprises an output device for outputting the test result, wherein a test result which is output via the output device is preferably input into the input interface for inputting the test result.

5. System according to any one of claims 1 to 4, **characterised in that** signalling device is configured for an optical and/or mechanical and/or acoustic display of the release and/or **in that** the release management system is additionally configured to withdraw release authorisations which have been set in the release database after a predetermined period of time has expired and/or after a predetermined time has been reached and/or **in that** the system is in the form of a distributed system, wherein components of the system can interact with each other via a network, preferably via the Internet.

6. System according to any one of claims 1 to 5, **characterised in that** the reading device for outputting the identification means is in the form of a barcode, a QR code - Quick Response Code, an RFID chip - Radio Frequency Identification Chip and/or an NFC identifier - Near Field Communication identifier.

7. System according to any one of claims 1 to 6, **characterised in that** the system has an identification database which associates the identification means and/or the identification marker encoded in the identification means with the person.

8. Method for identification protection in a restricted-access area, preferably using a system according to any one of claims 1 to 7, comprising the steps of:
taking a sample from a person who is located in the restricted-access area or who wishes to enter the restricted-access area,
associating the sample with an identification marker and/or
with the person in a sample database, testing the sample for the presence of at least one pathogen in the sample in order to obtain a test result,
setting a release authorisation in a release database when a comparison of the test result with a desired result provides a desired comparison result, wherein the release database is configured to associate the release authorisation with the identification marker and/or the person,
outputting an identification marker by means of a reading device in order to obtain an output identification marker, wherein the output identification marker is encoded in an identification means of the person,
outputting the release authorisation from the release database for the output identification marker, and
signalling a release of a restricted-access area by means of a signalling device when the release authorisation for the output identification marker is present, wherein the taking of a sample, the testing of the sample, and the setting of a release authorisation can be carried out on a regular basis, preferably daily.

9. Method according to claim 8, **characterised in that** the set release authorisation is deleted after a period of time has elapsed and/or when a time is reached.

10. Method according to claim 8 or 9, **characterised in that**, when the identification means is output to the person, the identification means and/or an identification marker encoded in the identification means is stored together with identification data of the person in an identification database.

11. Method according to any one of claims 8 to 10, **characterised in that** a sample marker is associated with a sample before or after the sample has been obtained, and **in that** the sample marker is associated with the identification marker and/or the person in a sample database.

12. Method according to any one of claims 8 to 11, **characterised in that**, before or when entering the restricted-access area, an identification marker which is encoded in the identification means is output by the reading device, **in that** for the output identification marker the release authorisation is requested from the release database and **in that**, when the release authorisation is present, access to the restricted-access area is allowed.

13. Method according to any one of claims 8 to 12, **characterised in that** the restricted-access area comprises a transit area, **in that** when leaving the restricted-access area the identification marker which is encoded in the identification means is output, **in that** for the output identification marker the release authorisation is requested from the release database and **in that** the restricted-access area can be left in a transit direction only when the release authorisation is present.

14. Method according to any one of claims 8 to 13, **characterised in that** entering and/or leaving the restricted-access area is detected in a protocol database, preferably in each case together with a time stamp and/or the output identification marker.

15. Method according to any one of claims 8 to 14, **characterised in that** quarantine measures can be initiated when the comparison of the test result with the desired result provides an undefined or undesirable comparison result.

## Revendications

1. Système de protection contre les infections dans une zone à accès restreint, comprenant :
un dispositif de test pour le test d'un échantillon afin de déterminer la présence d'au moins un agent pathogène dans l'échantillon, dans lequel au moins une partie de l'échantillon provient d'une personne qui se trouve dans la zone à accès restreint ou qui souhaite entrer dans la zone à accès restreint,
une interface d'entrée pour l'entrée d'un résultat de test qui a été obtenu grâce au dispositif de test au moyen du test de l'échantillon,
une base de données d'échantillons qui est conçue pour attribuer à l'échantillon un identifiant et/ou une personne,
une base de données de libération qui est conçue pour attribuer une autorisation de libération à l'identifiant et/ou à la personne,
un système de gestion de libération qui est conçu pour comparer le résultat de test entré dans l'interface d'entrée avec un résultat de consigne et, lors de l'obtention d'un résultat de test souhaité à l'aide de la base de données d'échantillons, pour régler l'autorisation de libération dans la base de données de libération,
un appareil de lecture pour l'obtention d'un identifiant lu par la lecture d'un identifiant codé dans un moyen d'identification de la personne,
un dispositif d'interrogation qui est conçu pour lire, sur la base de l'identifiant lu, une autorisation de libération présente ou non présente dans la base de données de libération et
un dispositif de signalisation qui est relié de manière communicante avec le dispositif d'interrogation et qui est conçu pour signaler, lors de la présence d'une autorisation de libération, une libération de la zone à accès restreint.

2. Système selon la revendication 1, **caractérisé en ce que** le système comprend un dispositif d'aliquotage et un dispositif de mélange, dans lequel le dispositif d'aliquotage est conçu pour tirer une aliquote de plusieurs échantillons individuels, dans lequel le dispositif de mélange est conçu pour mélanger les plusieurs aliquotes en un échantillon et dans lequel le dispositif de test effectue un test du mélange des plusieurs aliquotes, dans lequel le mélange des plusieurs aliquotes peut comprend plus de 10 aliquotes, de préférence plus de 20 aliquotes, plus particulièrement de préférence jusqu'à 100 aliquotes, encore plus particulièrement de préférence 32 aliquotes, et/ou dans lequel le système de gestion de libération est conçu pour comparer le résultat de test pour le mélange des plusieurs aliquotes avec le résultat de consigne et dans lequel le système de gestion de libération est en outre conçu pour régler, à l'aide de la base de données d'échantillons, l'autorisation de libération pour tous les échantillons individuels du mélange.

3. Système selon la revendication 1 ou 2, **caractérisé en ce que** le dispositif de test est conçu pour la réalisation d'une analyse par PCR - Polymerase Chain Reaction.

4. Système selon l'une des revendications 1 à 3, **caractérisé en ce que** le dispositif de test comprend un dispositif de sortie pour la sortie du résultat de test, dans lequel un résultat de test sorti grâce au dispositif de sortie est entré de préférence dans l'interface d'entrée pour l'entrée du résultat de test.

5. Système selon l'une des revendications 1 à 4, **caractérisé en ce que** le dispositif de signalisation est conçu comme un affichage optique et/ou mécanique et/ou acoustique de la libération et/ou **en ce que** le système de gestion de libération est en outre conçu pour extraire les autorisations de libération réglées dans la base de données de libération après l'écoulement d'une période prédéterminée et/ou après qu'un moment a été atteint et/ou **en ce que** le système est conçu comme un système réparti, dans lequel les composants du système peuvent interagir entre eux par l'intermédiaire d'un réseau, de préférence Internet.

6. Système selon l'une des revendications 1 à 5, **caractérisé en ce que** l'appareil de lecture est conçu pour la lecture du moyen d'identification sous la forme d'un code-barre, d'un QR-code - QUICK Response Code - d'une puce RFID - Radio Frequency Identification Chip - et/ou d'un identifiant NFC - Near Field Communication.

7. Système selon l'une des revendications 1 à 6, **caractérisé en ce que** le système comprend une base de données d'identification qui attribue à la personne le moyen d'identification et/ou l'identifiant codé dans le moyen d'identification.

8. Procédé de protection contre les infections dans une zone à accès restreint, de préférence à l'aide d'un système selon l'une des revendications 1 à 7, comprenant les étapes suivantes :
prise d'un échantillon par une personne qui se trouve dans la zone à accès restreint ou qui souhaite entrer dans la zone à accès restreint,
attribution de l'échantillon à un identifiant et/ou à la personne dans une base de données d'échantillons, test de l'échantillon afin de déterminer la présence d'au moins un agent pathogène dans l'échantillon afin d'obtenir un résultat de test,
réglage d'une autorisation de libération dans une base de données de libération lorsqu'une comparaison du résultat de test avec un résultat de consigne délivre un résultat de comparaison souhaité, dans lequel la base de données de libération est conçue pour attribuer à l'identifiant et/ou à la personne l'autorisation de libération,
lecture d'un identifiant au moyen d'un appareil de lecture afin d'obtenir un identifiant lu, dans lequel l'identifiant lu est codé dans un moyen d'identification de la personne,
lecture de l'autorisation de libération dans la base de données de libération pour l'identifiant lu et
signalisation d'une libération de la zone à accès restreint au moyen d'un dispositif de signalisation lors de la présence de l'autorisation de libération pour l'identifiant lu, dans lequel la prise d'un échantillon, le test de l'échantillon et le réglage d'une autorisation de libération peuvent être effectués régulièrement, de préférence quotidiennement.

9. Procédé selon la revendication 8, **caractérisé en ce que** l'autorisation de libération réglée est supprimée après l'écoulement d'une période et/ou après qu'un moment a été atteint.

10. Procédé selon la revendication 8 ou 9, **caractérisé en ce que**, lors de la sortie du moyen d'identification à l'attention de la personne, le moyen d'identification et/ou un identifiant codé dans le moyen d'identification est enregistré conjointement avec les données d'identification de la personne dans une base de données d'identification.

11. Procédé selon l'une des revendications 8 à 10, **caractérisé en ce qu'**à un échantillon est attribué un identifiant d'échantillon avant ou après l'obtention de l'échantillon et **en ce que** l'identifiant d'échantillon est attribué, dans une base de données d'échantillons, à l'identifiant et/ou à la personne.

12. Procédé selon l'une des revendications 8 à 11, **caractérisé en ce que**, avant ou après l'accès à la zone à accès restreint, un identifiant codé dans le moyen d'identification est lu au moyen de l'appareil de lecture, **en ce que**, pour l'identifiant lu, l'autorisation de libération est interrogée dans la base de données de libération et **en ce que**, lors de la présence de l'autorisation de libération, un accès à la zone à accès restreint est autorisé.

13. Procédé selon l'une des revendications 8 à 12, **caractérisé en ce que** la zone à accès restreint comprend une zone de transit, **en ce que**, lorsqu'on quitte la zone à accès restreint, l'identifiant codé dans le moyen d'identification est lu, **en ce que**, pour l'identifiant lu, l'autorisation de libération est interrogée dans la base de données de libération et **en ce que** la zone à accès restreint ne peut être quittée que dans une direction de transit lorsque l'autorisation de libération est présente.

14. Procédé selon l'une des revendications 8 à 13, **caractérisé en ce qu'**un accès et/ou une sortie de la zone à accès restreint est entré dans une base de données de protocoles, de préférence conjointement avec un horodatage et/ou l'identifiant lu.

15. Procédé selon l'une des revendications 8 à 14, **caractérisé en ce que** des mesures de quarantaine sont déclenchées lorsque la comparaison du résultat de test avec le résultat de consigne délivre un résultat de comparaison indéfini ou indésirable.
